(19) Europäisches Patentamt
European Patent Office
Office européen des brevets

(11) EP 4 613 364 A1

(12) **EUROPEAN PATENT APPLICATION**
published in accordance with Art. 153(4) EPC

(43) Date of publication:
10.09.2025 Bulletin 2025/37

(21) Application number: 23886182.7

(22) Date of filing: 30.10.2023

(51) International Patent Classification (IPC):
B01D 69/08 (2006.01)       B01D 69/12 (2006.01)
B01D 69/10 (2006.01)       A61M 1/16 (2006.01)
B01D 67/00 (2006.01)       B01D 71/68 (2006.01)

(52) Cooperative Patent Classification (CPC):
B01D 71/68; A61M 1/16; B01D 67/00; B01D 69/08;
B01D 69/088; B01D 69/10; B01D 69/12;
B01D 63/02; B01D 69/087; B01D 69/1212;
B01D 2323/18; B01D 2323/2185; B01D 2323/2187;
B01D 2323/219; B01D 2323/60;          (Cont.)

(86) International application number:
PCT/KR2023/017026

(87) International publication number:
WO 2024/096486 (10.05.2024 Gazette 2024/19)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 31.10.2022 KR 20220142641

(71) Applicant: INOSEP Inc
Gwangju 61005 (KR)

(72) Inventors:
• NGUYEN, Thanh Tin
  Gwangju 61005 (KR)
• YI, Eun Seong
  Gwangju 62274 (KR)
• LEE, Soo Han
  Gwangju 61967 (KR)
• HONG, Seung Jae
  Gwangju 62274 (KR)

(74) Representative: Marks & Clerk LLP
15 Fetter Lane
London EC4A 1BW (GB)

(54) **DOUBLE-LAYER HOLLOW FIBER MEMBRANE FOR HEMODIALYSIS AND METHOD FOR MANUFACTURING SAME**

(57) The present invention provides a double-layer hollow fiber membrane for enclosing a hollow body through which blood flows and delivering uremic toxins and waste products in the blood to the outside, and a method for manufacturing same. The double-layer hollow fiber membrane comprises a first layer having first pores and a second layer having second pores. The first pores of the first layer and the second pores of the second layer are connected to each other, and the second pores are open pores connected to the outside. The double-layer hollow fiber membrane can remove mid-molecular-sized uremic toxins in the blood while minimizing the loss of blood components and essential proteins due to the pores whose size increases from the first pores to the second pores.

FIG. 7

Measurement of Mass of Dried Membrane → Immersion in IPA for 24 Hours → Measurement of Mass of Wetted Membrane

(52) Cooperative Patent Classification (CPC): (Cont.)
B01D 2325/0283

**Description**

[Technical Field]

The present invention relates to a double-layer hollow fiber membrane, and more specifically, to a double-layer hollow fiber membrane that can transfer uremic toxins and waste products in blood to the outside, while minimizing the loss of essential blood components and vital proteins, as well as to a method for manufacturing the same.

[Background Art]

Kidney disease is a condition in which fluids and waste products that should be excreted from the patient's body accumulate in the bloodstream due to impaired kidney function, leading to an imbalance of electrolytes in the body. Chronic kidney disease refers to such conditions that persist for more than three months, and result in proteinuria or hematuria due to kidney damage or impaired function. The number of patients with chronic kidney disease is steadily increasing worldwide, and in Korea as well, the patient population continues to grow each year due to rapid population aging and the growing prevalence of risk factors such as hypertension, diabetes, and metabolic syndrome.

Renal replacement therapies, such as hemodialysis, peritoneal dialysis, and kidney transplantation, are used to remove waste products from the bodies of patients with chronic kidney disease. According to the results of a survey conducted in 2022, 79.4% of patients receiving renal replacement therapy underwent hemodialysis, 4.1% received peritoneal dialysis, and 16.5% underwent kidney transplantation, indicating that the majority of patients are treated with hemodialysis (Non-Patent Document 1).

Hemodialysis (HD) is a treatment method that removes accumulated impurities from the body by circulating the patient's blood outside the body. It operates on the principle of diffusion, which occurs due to concentration gradients between blood and dialysate, to remove uremic toxins and waste products from the blood through a semipermeable membrane known as a hemodialysis filter, after which the purified blood is returned to the body. The hemodialysis method removes uremic toxins and excess fluid from the body by circulating blood on one side of a semipermeable membrane and dialysate on the other. However, conventional hemodialysis primarily removes low-molecular-weight uremic toxins through diffusion driven by concentration gradients. As a result, large middle-molecular-weight uremic toxins, with molecular weights exceeding 1 kDa, are not effectively removed, highlighting the need for a new hemodiafiltration method.

Hemodiafiltration (HDF) is a treatment method that combines hemodialysis with filtration. While hemodiafiltration is similar to hemodialysis in that it uses a dialysis membrane, it differs by enhancing convective transport of solutes, which allows for the removal of middle-molecular-weight waste products (such as urea). Moreover, various clinical studies have demonstrated that hemodiafiltration results in higher patient survival rates compared to conventional hemodialysis. However, current hemodiafiltration still exhibits lower clearance of uremic toxins and waste products compared to healthy kidneys, and concerns remain regarding the potential loss of essential blood components due to pressurized filtration.

Meanwhile, hollow fiber membranes are fiber-like separation membranes that contain a hollow core. In therapeutic blood treatment, hollow fiber membranes are used as separation membranes aimed at removing waste products from the blood. Materials commonly used for hemodialysis membranes are primarily polymer-based, including cellulose-based, cellulose acetate-based, polyamide-based, polyolefin-based, polyacrylonitrile-based, and polysulfone-based materials. Among these, polysulfone-based materials and polyvinylpyrrolidone (PVP), known for their excellent blood compatibility, are becoming increasingly widespread. However, if a significant amount of PVP remains in the hollow fiber membrane, it may be released during hemodialysis, potentially leading to adverse effects.

Various methods are used to manufacture hollow fiber membranes, including phase inversion, thermally induced phase separation (TIPS), melt spinning, dry-wet spinning, and stretching methods, with the phase inversion method being the most commonly used. However, the phase inversion method leads to irregular positioning and sizes of pores during the phase transition process, resulting in poor filtration precision and capacity.

Therefore, there is a need for the development of hollow fiber membranes that not only have excellent water permeability but also can remove middle-molecular-weight uremic toxins, which are critical for long-term dialysis patients.

[Prior Art Documents]

[Patent Documents]

Korean Patent No. 10-1810470 (Dec. 13, 2017)
Korean Patent Application Publication No. 10-2005-0078748 (Aug. 8, 2005)

[Non-Patent Documents]

Non-Patent Document 1: "Current Status of Renal Replacement Therapies in Korea, 2023," the Korean Society of Nephrology

[Disclosure]

[Technical Problem]

To address the aforementioned issues, a first object of the present invention is to provide a double-layer hollow fiber membrane that surrounds a hollow core through which blood flows and includes pores with controlled sizes to effectively remove uremic toxins from the blood.

A second object of the present invention is to provide a method for manufacturing the double-layer hollow fiber membrane of the first object by adjusting the content of a water-soluble pore-forming polymer.

[Technical Solution]

To achieve the first object, the present invention provides a hollow fiber membrane that surrounds a hollow core through which blood flows and is configured to transfer uremic toxins and waste products in the blood flowing through the hollow core to the outside. The double-layer hollow fiber membrane may comprise a first layer having first pores and a second layer connected to the first layer and having second pores that are larger in size than the first pores. The second pores of the second layer are connected to the first pores of the first layer and are in the form of open pores that are also connected to the outside.

To achieve the second problem, the present invention provides a method for manufacturing a double-layer hollow fiber membrane using a spinning solution in which a water-soluble pore-forming polymer is dissolved in a solvent. The water-soluble pore-forming polymer may be a polyvinyl-based resin, a glycol-based resin, or a mixed resin thereof. Specifically, the double-layer hollow fiber membrane of the first object may be manufactured by supplying a bore solution, a first spinning solution prepared by dissolving a polyvinyl-based resin and a polysulfone-based resin in a solvent, and a second spinning solution prepared by dissolving a glycol-based resin and a polysulfone-based resin in a solvent to a triple nozzle, spinning the solutions into a coagulation bath, taking up the spun fiber using a winder to form a hollow fiber membrane, gelling the membrane in a water bath, washing the resulting membrane in a washing bath containing 80 wt% to 100 wt% of water and 0 wt% to 20 wt% of glycerol, and drying the resulting membrane in the air at room temperature.

[Advantageous Effects]

The double-layer hollow fiber membrane of the present invention can remove uremic toxins and waste products from blood flowing through the hollow core. The blood flowing through the hollow core comes into contact with the first layer of the hollow fiber membrane, and through such contact, the uremic toxins and waste products in the blood can migrate into the first pores of the first layer. The uremic toxins and waste products that have migrated from the blood can further migrate into the second pores of the second layer of the hollow fiber membrane, which are connected to the first pores. The second layer comes into contact with dialysate, and through such contact, the uremic toxins and waste products in the blood can diffuse into the dialysate through the second pores connected to the first pores.

The second pores are connected to the first pores, allowing uremic toxins and waste products that have passed through the first pores to diffuse into the dialysate without obstruction. Furthermore, since the second pores are larger in size than the first pores, the contact area between the uremic toxins and waste products, which have passed through the first pores, and the dialysate can be increased, thereby providing a faster diffusion pathway.

In addition, the first pores of the double-layer hollow fiber membrane of the present invention are suitable for removing middle-molecular-weight uremic toxins, such that essential proteins and albumin, which are larger than the uremic toxins, are not filtered and can be maintained at a safe level, thereby enabling the membrane to be used as a separation membrane for hemodialysis, hemofiltration, or hemodiafiltration.

[Description of Drawings]

FIG. 1 is an enlarged image of a triple nozzle used in a method for manufacturing a double-layer hollow fiber membrane of the present invention.
FIG. 2 is a conceptual diagram illustrating a process for forming open pores through the triple nozzle in the method for manufacturing the double-layer hollow fiber membrane of the present invention.

FIG. 3 is a set of graphs illustrating the average values of the outer diameter, inner diameter, membrane thickness, and porosity of the double-layer hollow fiber membrane manufactured by the method of the present invention.

FIG. 4 is a set of images illustrating a process for forming a mini-module comprising the double-layer hollow fiber membrane manufactured by the method of the present invention.

FIG. 5 is a set of SEM images showing the cross-sections of the double-layer hollow fiber membranes manufactured by the method of the present invention.

FIG. 6 is a set of SEM images showing the first and second pores in the form of open pores of the double-layer hollow fiber membrane manufactured by the method of the present invention.

FIG. 7 is a flowchart for measuring the porosity of the double-layer hollow fiber membrane manufactured by the method of the present invention.

FIG. 8 is a schematic diagram of a filtration apparatus for evaluating the pure water permeability of the double-layer hollow fiber membrane using a mini-module comprising the double-layer hollow fiber membrane manufactured by the method of the present invention.

FIG. 9 is a set of graphs illustrating the water permeability of the double-layer hollow fiber membranes manufactured by the method of the present invention.

FIG. 10 is a schematic diagram of a filtration apparatus for evaluating the bovine serum albumin leakage of the hollow fiber membrane using a mini-module comprising the double-layer hollow fiber membrane manufactured by the method of the present invention.

FIG. 11 is a set of graphs illustrating the bovine serum albumin leakage of the double-layer hollow fiber membranes manufactured by the method of the present invention.

FIG. 12 is a schematic diagram of a filtration apparatus for evaluating the molecular weight cut-off of the hollow fiber membrane using a mini-module comprising the double-layer hollow fiber membrane manufactured by the method of the present invention.

FIG. 13 is a set of graphs illustrating the pore size, standard deviation of pore size, and molecular weight cut-off of the double-layer hollow fiber membranes manufactured by the method of the present invention.

FIG. 14 is a schematic diagram of a filtration apparatus for evaluating the hemodialysis performance of the hollow fiber membrane using a mini-module comprising the double-layer hollow fiber membrane manufactured by the method of the present invention.

FIG. 15 is a set of graphs illustrating the bovine serum albumin leakage and the removal rates of urea and creatinine of the double-layer hollow fiber membrane manufactured by the method of the present invention.

[Mode for Carrying Out the Invention]

Hereinafter, embodiments of the present invention will be described in detail with reference to the accompanying drawings so that those skilled in the art to which the invention pertains can easily carry out the invention. These embodiments of the present invention are provided to more completely describe the invention to those skilled in the art. Thus, the embodiments of the present invention can be modified in various forms, and the scope of the present invention is not limited to the embodiments described below, but can be realized in other forms.

Throughout the specification of the present invention, when it is said that a certain part "includes" a certain component, it means that it may further include other components without excluding other components, unless specifically stated otherwise.

Throughout the specification of the present invention, the term "step of ~ing" or "~ing step" should not be interpreted as "step for ~ing."

As used herein, the term "membrane" refers to a discrete, generally thin interface that moderates the permeation of chemical species in contact with it, and this membrane includes pores of finite dimensions as defined below.

**EXAMPLES**

The present invention provides a double-layer hollow fiber membrane that surrounds a hollow core through which blood flows and is configured to transfer uremic toxins and waste products in the blood flowing through the hollow core to the outside. The double-layer hollow fiber membrane may comprise a first layer having first pores and a second layer connected to the first layer and having second pores that are larger in size than the first pores. The second layer having the second pores can be in contact with dialysate.

In the double-layer hollow fiber membrane, the first pores of the first layer and the second pores of the second layer may be connected to allow uremic toxins and waste products in the blood flowing through the hollow core to pass through. Moreover, the second pores of the second layer may be in the form of open pores connected to the outside to allow the uremic toxins and waste products in the blood to diffuse into the dialysate. In the double-layer hollow fiber membrane, the pore size of the first pores may be smaller than that of blood components, such as red blood cells, platelets, and white blood cells, and essential proteins such as albumin, yet larger than middle-molecular-weight uremic toxins and waste products. Specifically, the pore size of the first pores may range from 5 nm to 12 nm, with a preferred range of 8 nm to 11 nm. In the double-layer hollow fiber membrane, the pore size of the second pores may be larger than that of the first pores. Specifically, the pore size of the second pores may range from 0.1 $\mu$m to 1.5 $\mu$m, with a preferred range of 0.3 $\mu$m to 1.0 $\mu$m.

In the above double-layer hollow fiber membrane, the first pores of the first layer and the second pores of the second layer may have different sizes formed using different types of water-soluble pore-forming polymers. Furthermore, the size and uniformity of the pores and the degree of formation of open pores may be controlled by varying the composition of the water-soluble pore-forming polymer. The pore-forming agent may be a polyvinyl-based resin or a glycol-based resin. Specifically, the first pores of the first layer may be formed using a polyvinyl-based resin, while the second pores of the second layer may be formed using a glycol-based resin. The polyvinyl-based resin forming the first pores of the first layer may be induced to diffuse toward the outer membrane side of the second layer due to the diffusion effect of the glycol-based resin forming the second pores of the second layer, and may affect the formation of the second pores in the form of open pores formed by the diffusion induction. The glycol-based resin forming the second pores of the second layer may affect the size of the first pores due to its relatively high content and low molecular weight. In addition, the polyvinyl-based resin of the first layer and the glycol-based resin of the second layer may affect the size and uniformity of the first and second pores through adjustment of their respective contents.

The polyvinyl-based resin as the water-soluble pore-forming polymer may comprise at least one selected from the group consisting of polyvinylpyrrolidone (PVP), polyvinylacetate (PVAc), polyvinylalcohol (PVA), and polyethylene-co-vinylacetate (PEVA), and may specifically be PVP. The glycol-based resin as the water-soluble pore-forming polymer may comprise at least one selected from the group consisting of ethylene glycol (EG), diethylene glycol (DEG), triethylene glycol (TEG), propylene glycol (PG), and polyethylene glycol (PEG), and may specifically be PEG.

The double-layer hollow fiber membrane may have a water permeability of 120 LMH·bar$^{-1}$ to 330 LMH·bar$^{-1}$, and preferably 150 LMH·bar$^{-1}$ to 300 LMH·bar$^{-1}$. If the permeability of the hollow fiber membrane is less than 120 LMH·bar$^{-1}$, the filtration efficiency of uremic toxins and waste products in blood may be deteriorated, whereas if it is greater than 330 LMH·bar$^{-1}$, it may lead to a loss of essential blood components and vital proteins.

The double-layer hollow fiber membrane may be used as a separation membrane for hemodialysis, hemofiltration, or hemodiafiltration to filter uremic toxins and waste products from blood. The uremic toxins or waste products to be filtered may comprise at least one selected from the group consisting of urea, uric acid, beta-2 microglobulin, lambda free light chain, kappa free light chain, creatinine, hippuric acid, indoxyl sulfate, p-cresol, oxalates, guanidines, phenol, and homocysteine. The double-layer hollow fiber membrane may be used for the purpose of improving at least one kidney disease selected from the group consisting of diabetic nephropathy, chronic kidney failure, acute kidney failure, subacute kidney failure, glomerulonephritis, malignant nephrosclerosis, vascular microangiopathy, transplant rejection, glomerulopathy, kidney hypertrophy, kidney hyperplasia, proteinuria, contrast-induced nephropathy, toxin-induced kidney injury, oxygen free-radical-mediated nephropathy, and nephritis, by removing uremic toxins or waste products from the blood.

Additionally, the present invention provides a method for manufacturing a double-layer hollow fiber membrane using a spinning solution comprising a water-soluble pore-forming polymer. Specifically, the present invention provides a method for manufacturing a double-layer hollow fiber membrane using a first spinning solution containing a first water-soluble pore-forming polymer and a polysulfone-based resin and a second spinning solution containing a second water-soluble pore-forming polymer and a polysulfone-based resin. The first spinning solution may be prepared by dissolving a first water-soluble pore-forming polymer and a polysulfone-based resin in a solvent, and the second spinning solution may be prepared by dissolving a second water-soluble pore-forming polymer and a polysulfone-based resin in a solvent. The double-layer hollow fiber membrane may be formed by supplying the first spinning solution and the second spinning solution simultaneously with a bore solution to a triple nozzle, spinning the solutions, and then taking up the spun fiber using a winder. The double-layer hollow fiber membrane may be manufactured by gelling the resulting membrane in a water bath, washing the resulting membrane in a washing bath containing 80 wt% to 100 wt% of water and 0 wt% to 20 wt% of glycerol, and drying the resulting membrane in the air at room temperature.

In the manufacturing method, the temperature of the triple nozzle may be 40 °C to 55 °C. In the manufacturing method, the bore solution may be supplied to a first nozzle, which is at the innermost center of the triple nozzle; the first spinning solution may be supplied to a second nozzle, which is provided on the outer circumference of the first nozzle; and the second spinning solution may be supplied to a third nozzle, which is provided on the outer circumference of the second nozzle. The flow rate of the bore solution supplied to the first nozzle may range from 0.1 ml/min to 1.5 ml/min, and specifically may range from 0.3 ml/min to 1.0 ml/min. The flow rate of the first and second spinning solutions supplied to the second and third nozzles may range from 0.1 ml/min to 1.0 ml/min, and specifically may range from 0.1 ml/min to 0.5 ml/min.

In the manufacturing method, the bore solution may act as an internal coagulant and contribute to the formation of the double-layer hollow fiber membrane. The bore solution may comprise at least one selected from the group consisting of water, glycerol, N-methyl-2-pyrrolidone, a glycol-based resin, an alcohol-based solvent, and a ketone-based solvent.

Specifically, it may comprise at least one selected from the group consisting of water, glycerol, dimethoxyethanol, N-methyl-2-pyrrolidone, methanol, ethanol, isopropanol, acetone, and dimethylacetamide. Preferably, it may be glycerol dissolved in a solvent, and more preferably, glycerol dissolved in N-methyl-2-pyrrolidone.

The solvent may be an aprotic polar solvent, and the aprotic polar solvent may be at least one solvent selected from the group consisting of acetone, acetonitrile, dimethyl acetamide (DMAc), dimethyl formamide (DMF), dimethyl sulfoxide (DMSO), methyl ethyl ketone, methyl n-propyl ketone, N-methyl-2-pyrrolidone, propylene carbonate, nitromethane, sulforane, and hexamethylphosphoramide (HMP). Preferably, it may be N-methyl-2-pyrrolidone.

The first spinning solution may comprise a polyvinyl-based resin, a polysulfone-based resin, and N-methyl-2-pyrrolidone. Specifically, the first spinning solution may comprise 1 wt% to 20 wt% of polyvinyl-based resin, 10 wt% to 20 wt% of polysulfone-based resin, and 65 wt% to 85 wt% of N-methyl-2-pyrrolidone. The polyvinyl-based resin is a water-soluble resin that can form uniform water channels within the support layer and can affect the size of the water channels formed in the first and second layers of the double-layer hollow fiber membrane being manufactured. If the polyvinyl-based resin is included in an amount of less than 1 wt%, the solvent may dissociate in the first spinning solution, leading to a reduced solidification rate. This reduced solidification rate can cause an increase in the size of the pores formed, allowing blood components and vital proteins to diffuse into the dialysate. Moreover, if the polyvinyl-based resin exceeds 20 wt%, the pore size may become excessively large, allowing blood components and vital proteins such as albumin to diffuse into the dialysate. Therefore, it is preferable for the polyvinyl-based resin to be included in an amount of 1 wt% to 20 wt%.

The second spinning solution may comprise a glycol-based resin, a polysulfone-based resin, and N-methyl-2-pyrrolidone. Specifically, the second spinning solution may comprise 5 wt% to 30 wt% of glycol-based resin, 10 wt% to 20 wt% of polysulfone-based resin, and 50 wt% to 80 wt% of N-methyl-2-pyrrolidone. The glycol-based resin is a water-soluble resin that can form uniform water channels within the support layer and can affect the formation and size of the water channels of the double-layer hollow fiber membrane being manufactured. If the glycol-based resin is included in an amount of less than 5 wt%, the pore size may not sufficiently increase, resulting in a reduction in water permeability, whereas if the glycol-based resin exceeds 20 wt%, the pore size may become excessively large, allowing blood components and vital proteins to diffuse into the dialysate. Therefore, it is preferable for the glycol-based resin to be included in an amount of 5 wt% to 20 wt%.

In the manufacturing method, the polysulfone-based resin included in the first and second spinning solutions may be used as a membrane material due to its excellent chemical resistance, mechanical properties, biocompatibility, and cost-effectiveness. If the polysulfone-based resin is included in an amount of less than 10 wt%, the mechanical properties may deteriorate, whereas if it exceeds 20 wt%, the thickness of the hollow fiber membrane may increase, thereby reducing the removal efficiency of uremic toxins and waste products from the blood. Therefore, it is preferable for the polysulfone-based resin to be included in an amount of 10 wt% to 20 wt%. Specifically, the polysulfone-based resin may be at least one resin selected from the group consisting of polyethersulfone (PES), polysulfone (PSU), and polyphenylenesulfone (PPSU). Preferably, it may preferably be PES, which exhibits high hydrophilicity, reduces membrane fouling caused by organic substances, and is bisphenol-A free.

In the manufacturing method, a step of supplying an inert gas to remove impurities may be additionally performed after preparing the first and second spinning solutions. The inert gas may be at least one selected from the group consisting of neon, argon, nitrogen, and helium.

Since the types of the polyvinyl-based resin and glycol-based resin included in the first and second spinning solutions in the manufacturing method are the same as those described above in connection with the double-layer hollow fiber membrane, the foregoing description is incorporated herein by reference.

In the manufacturing method, the coagulation bath into which the bore solution, the first spinning solution, and the second spinning solution are spun may contain an external coagulant. The external coagulant may be used without limitation as long as it can substitute for the bore solution, which is the internal coagulant. Specifically, it may be a solvent or a non-solvent, and preferably, a non-solvent containing a small amount of solvent.

The non-solvent may be water, a glycol-based resin, a $C_1$ to $C_1$ alcohol or a mixture thereof, and the solvent may be glycerol, N-methyl-2-pyrrolidone, or a ketone-based solvent. The temperature of the coagulation bath may be a temperature at which phase transition is facilitated, and specifically, may be 10 °C to 30 °C, and preferably from 10 °C to 25 °C.

In the manufacturing method, the air gap between the triple nozzle and the coagulation bath may be 15 cm to 180 cm, and preferably from 25 cm to 100 cm. If the length of the air gap is less than 15 cm, the spinning solution may not be sufficiently elongated before coagulation, which may make it difficult to control the dimensions (i.e., outer diameter, inner diameter, thickness, and pore size) of the hollow fiber membrane. Moreover, if the length of the air gap exceeds 180 cm, the elongated spinning solution may be exposed for a longer time to an external environment that is difficult to control, which may adversely affect the formation of a hollow fiber membrane with a uniform structure, leading to unexpected defects.

In the manufacturing method, the atmospheric temperature between the triple nozzle and the coagulation bath may be 15 °C to 120 °C, and preferably from 20 °C to 100 °C. Furthermore, the atmospheric humidity between the triple nozzle and the external coagulant may be 45% to 55%, and preferably from 45% to 80%.

In the manufacturing method, the winder for taking up the formed hollow fiber membrane may operate at a speed of 7 m/min to 25 m/min, and preferably at a speed of 10 m/min to 20 m/min. If the take-up speed is less than 7 m/min, uneven extrusion of the solution may occur, which may result in structural damage to the membrane. In addition, if the take-up speed exceeds 25 m/min, the pore size may become excessively large, potentially causing the leakage of useful proteins such as albumin from the blood. Furthermore, the spinning solution may fail to keep up with the take-up speed, resulting in defects or breakage during continuous processing. Therefore, the take-up speed of the winder may be 7 m/min to 25 m/min.

Hereinafter, the present invention will be described in detail with reference to the following Preparation Examples and Experimental Examples.

**Preparation Example 1: Preparation of the First Spinning Solutions**

**1-1. Preparation of the First-First Spinning Solution**

280 g of polyethersulfone (PES) and 100 g of polyvinylpyrrolidone (PVP) were placed in a 2 L round-bottom flask containing N-methyl-2-pyrrolidone (NMP) solvent.

The resulting mixture was stirred at 60 °C and 200 rpm for one day, followed by degassing at room temperature to prepare the first-first spinning solution. The first-first spinning solution prepared was then purged with nitrogen at a pressure of 18 psi to remove impurities.

**1-2. Preparation of the First-Second Spinning Solution**

The first-second spinning solution was prepared in the same manner as described in Preparation Example 1-1, except that 280 g of PES and 200 g of PVP were added to the NMP solvent.

**1-3. Preparation of the First-Third Spinning Solution**

The first-third spinning solution was prepared in the same manner as described in Preparation Example 1-1, except that 280 g of PES and 50 g of PVP were added to the NMP solvent.

**Preparation Example 2: Preparation of the Second Spinning Solutions**

**2-1. Preparation of the Second-First Spinning Solution**

280 g of PES and 400 g of polyethylene glycol (PEG) were placed in a 2 L round-bottom flask containing NMP solvent.

The resulting mixture was stirred at 60 °C and 200 rpm for one day, followed by degassing at room temperature to prepare the second-first spinning solution. The prepared second-first spinning solution was then purged with nitrogen at a pressure of 18 psi to remove impurities.

**2-2. Preparation of the Second-Second Spinning Solution**

The second-second spinning solution was prepared in the same manner as described in Preparation Example 2-1, except that 280 g of PES and 560 g of PEG were added to the NMP solvent.

**2-3. Preparation of the Second-Third Spinning Solution**

The second-third spinning solution was prepared in the same manner as described in Preparation Example 2-1, except that 280 g of PES and 240 g of PEG were added to the NMP solvent.

**2-4. Preparation of the Second-Fourth Spinning Solution**

The second-fourth spinning solution was prepared in the same manner as described in Preparation Example 2-1, except that 280 g of PES and 20 g of PEG were added to the NMP solvent.

**Preparation Example 3: Preparation of Bore Solution**

Deionized water (DI-water) and 100 g of glycerol were mixed in NMP solvent. The resulting mixture was stirred at 180 rpm for 1 hour to prepare the bore solution.

The prepared bore solution was stored in a brown bottle at 45 °C.

**Preparation Example 4: Preparation of Double-Layer Hollow Fiber Membranes**

**4-1. Preparation of the First Double-Layer Hollow Fiber Membrane**

The first-first spinning solution prepared in Preparation Example 1-1, the second-first spinning solution prepared in Preparation Example 2-1, and the bore solution prepared in Preparation Example 3 were supplied to a triple nozzle to prepare a double-layer hollow fiber membrane.

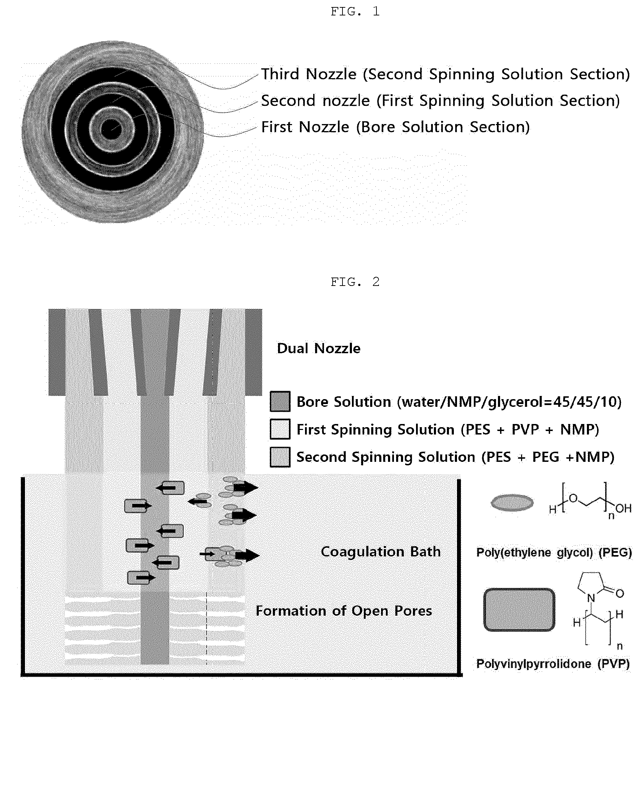

Referring to FIG. 1, the triple nozzle comprises a first nozzle, which is at the innermost center, a second nozzle, which is provided on the outer circumference of the first nozzle, and a third nozzle, which is provided on the outer circumference of the second nozzle. Referring to FIG. 2, the double-layer hollow fiber membrane was prepared by supplying the first spinning solution, the second spinning solution, and the bore solution to the triple nozzle.

Specifically, a triple nozzle was prepared with an air gap of 25 cm between the nozzle and the coagulation bath, and nozzle temperature was maintained at 50 °C (atmospheric temperature: 20 °C to 30 °C; relative humidity: 40% to 50%). The first-first spinning solution, the second-first spinning solution, and the bore solution were allowed to stand at room temperature for 12 hours to remove air bubbles. The triple nozzle was then simultaneously supplied with the bore solution to the first nozzle at a flow rate of 0.7 mL/min, the first spinning solution to the second nozzle at a flow rate of 0.333 mL/min (1 rpm), and the second spinning solution to the third nozzle at a flow rate of 0.333 mL/min (1 rpm).

The supplied first spinning solution, second spinning solution, and bore solution were simultaneously spun into a coagulation bath filled with water at 25 °C, and the spun fiber was taken up at a speed of 13 m/min using a winder to prepare a hollow fiber membrane. The prepared hollow fiber membrane was subjected to gelation in a water bath, washed with water, and subsequently stored in glycerol.

As a result, a double-layer hollow fiber membrane was obtained, with an outer diameter of 298.1 $\mu$m, an inner diameter of 229.8 $\mu$m, and a thickness of 33.2 $\mu$m.

**4-2. Preparation of the Second Double-Layer Hollow Fiber Membrane**

A double-layer hollow fiber membrane was prepared in the same manner as described in Preparation Example 4-1, except that the first-first spinning solution prepared in Preparation Example 1-1, the second-second spinning solution prepared in Preparation Example 2-2, and the bore solution prepared in Preparation Example 3 were supplied to the triple nozzle.

As a result, a double-layer hollow fiber membrane was obtained, with an outer diameter of 284.6 $\mu$m, an inner diameter of 221.2 $\mu$m, and a thickness of 33.8 $\mu$m.

**4-3. Preparation of the Third Double-Layer Hollow Fiber Membrane**

A double-layer hollow fiber membrane was prepared in the same manner as described in Preparation Example 4-1, except that the first-first spinning solution prepared in Preparation Example 1-1, the second-third spinning solution prepared in Preparation Example 2-3, and the bore solution prepared in Preparation Example 3 were supplied to the triple nozzle.

As a result, a double-layer hollow fiber membrane was obtained, with an outer diameter of 286.9 $\mu$m, an inner diameter of 223.5 $\mu$m, and a thickness of 34.5 $\mu$m.

**4-4. Preparation of the Fourth Double-Layer Hollow Fiber Membrane**

A double-layer hollow fiber membrane was prepared in the same manner as described in Preparation Example 4-1, except that the first-second spinning solution prepared in Preparation Example 1-2, the second-first spinning solution prepared in Preparation Example 2-1, and the bore solution prepared in Preparation Example 3 were supplied to the triple nozzle.

As a result, a double-layer hollow fiber membrane was obtained, with an outer diameter of 288.2 $\mu$m, an inner diameter of 214.9 $\mu$m, and a thickness of 35.9 $\mu$m.

**4-5. Preparation of the Fifth Double-Layer Hollow Fiber Membrane**

A double-layer hollow fiber membrane was prepared in the same manner as described in Preparation Example 4-1, except that the first-third spinning solution prepared in Preparation Example 1-3, the second-first spinning solution prepared in Preparation Example 2-1, and the bore solution prepared in Preparation Example 3 were supplied to the triple nozzle.

As a result, a double-layer hollow fiber membrane was obtained, with an outer diameter of 290.0 $\mu$m, an inner diameter of 227.5 $\mu$m, and a thickness of 32.6 $\mu$m.

### 4-6. Preparation of the Sixth Double-Layer Hollow Fiber Membrane

A double-layer hollow fiber membrane was prepared in the same manner as described in Preparation Example 4-1, except that the first-second spinning solution prepared in Preparation Example 1-2, the second-fourth spinning solution prepared in Preparation Example 2-4, and the bore solution prepared in Preparation Example 3 were supplied to the triple nozzle.

As a result, a double-layer hollow fiber membrane was obtained, with an outer diameter of 282.8 $\mu$m, an inner diameter of 217.6 $\mu$m, and a thickness of 35.7 $\mu$m.

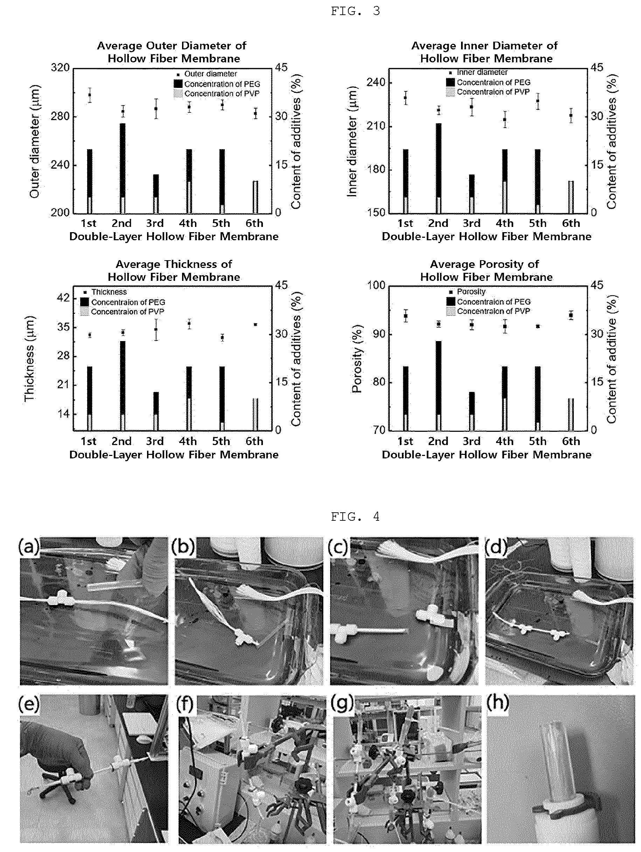

Referring to FIG. 3, it was confirmed that the average outer diameter, average inner diameter, average membrane thickness, and average porosity of the first to sixth double-layer hollow fiber membranes prepared in Preparation Examples 4-1 to 4-6 were within the typical range of hollow fiber membranes, indicating that the spinning solutions had little effect on the outer diameter, inner diameter, membrane thickness, and porosity of the hollow fiber membranes.

### Preparation Example 5: Fabrication of Double-Layer Hollow Fiber Membrane Mini-Modules

Perfluoroalkoxy alkane (PFA) tubing with an inner diameter of 3.98 mm and an outer diameter of 6.4 mm was cut to obtain an effective membrane length of 9.5 cm. T-type one-touch fitting was used to fabricate the module housing, allowing separation between the blood section and the dialysate section.

To achieve a packing density of approximately 10%, 18 to 20 strands of the first to sixth double-layer hollow fiber membranes prepared in Preparation Examples 4-1 to 4-6 were cut to a length of 250 mm and assembled into the tubing housing. Both ends of the tubing housing were potted and then dried for 12 hours. After drying, the epoxy injection sections were trimmed to complete the fabrication of the mini-modules.

The number of strands used for the first to sixth double-layer hollow fiber membranes prepared in Preparation Examples 4-1 to 4-6 and the effective surface area of the fabricated modules are shown in Table 1 below:

[Table 1]

| | First Double-Layer Hollow Fiber Membrane | Double-Layer Second Hollow Fiber Membrane | Third Double-Layer Hollow Fiber Membrane | Fourth Double-Layer Hollow Fiber Membrane | Fifth Double-Layer Hollow Fiber Membrane | Sixth Double-Layer Hollow Fiber Membrane |
|---|---|---|---|---|---|---|
| Tubing Inner Diameter (mm) | 3.98 | 3.98 | 3.98 | 3.98 | 3.98 | 3.98 |
| Number of Strands (n) | 18 (17.826) | 20 (19.557) | 20 (19.2444) | 20 (19.071) | 19 (18.835) | 20 (19.806) |
| Effective Surface Area (m$^2$) | 0.001234514 | 0.001320349 | 0.001334077 | 0.001282744 | 0.001290056 | 0.001298860 |

The packing density was calculated using Equation 1 below:

[Equation 1]

$$\text{Packing Density (\%)} = 100 \times n \times \frac{r^2}{R^2}$$

(In the above Equation, n is the number of strands of the hollow fiber membranes, r is the outer radius of a single hollow fiber membrane, and R is the inner radius of the tubing.)

Referring to FIG. 4, the fabrication of the mini-module using the double-layer hollow fiber membranes involves the following steps: selecting and cutting the hollow fiber membranes (FIG. 4, Steps (a) to (d)); assembling the tubing housing and the hollow fiber membranes (FIG. 4, Step (e)); injecting epoxy into both ends of the assembled tubing housing, followed by drying (FIG. 4, Steps (f) to (g)); and cutting the dried epoxy (FIG. 4, Step (h)).

**Experimental Example 1: Structural Analysis of Double-Layer Hollow Fiber Membranes**

The first to sixth double-layer hollow fiber membranes prepared in Preparation Examples 4-1 to 4-6 were cut in half under a nitrogen solution and then coated with platinum (Pt) for 3 minutes.

The cross-section ($\times$200) and side surface ($\times$1,500) of the coated double-layer hollow fiber membranes, the cross-section ($\times$10,000) and membrane surface ($\times$500) of the second layer of the double-layer hollow fiber membrane, and the cross-section ($\times$10,000) and membrane surface ($\times$100,000) of the first layer of the double-layer hollow fiber membrane were analyzed using a scanning electron microscope (SEM).

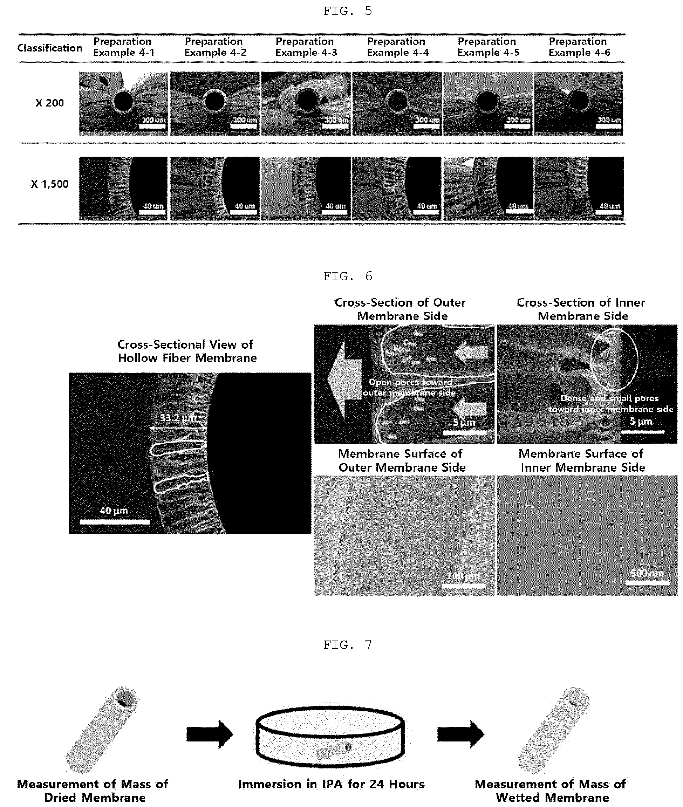

Referring to FIGS. 5 and 6, SEM analysis of the cross-section of the hollow fiber membranes prepared by the manufacturing method of the present invention confirmed that the double-layer hollow fiber membranes have a double-layer structure, comprising a first layer containing first pores and a second layer containing second pores.

Referring to FIG. 6, analysis of the cross-sections of the first and second layers of the double-layer hollow fiber membrane of the present invention revealed a distinct difference in pore size between the first and second pores, with the pores increasing in size from the first layer toward the second layer.

Moreover, it was confirmed that the first and second pores are connected, and that the second pores are open pores capable of allowing substances to pass through to the outside. A distinct difference in pore size was also observed between the membrane surfaces of the first and second layers of the double-layer hollow fiber membrane.

**Experimental Example 2: Porosity Test of Double-Layer Hollow Fiber Membranes**

The first to sixth double-layer hollow fiber membranes prepared in Preparation Examples 4-1 to 4-6 were dried in an oven at 50 °C for one day and then cut into 10 cm lengths to prepare bundles. After measuring the mass of the bundles, the bundles used for mass measurement were immersed in isopropanol (IPA) for 24 hours.

After the immersion, the bundles were taken out, and the IPA on the surface and inside the hollow fibers was removed. The mass of the wetted hollow fiber membrane bundles, with IPA infiltrated into the pores, was then measured to calculate the porosity.

The porosity was calculated using Equation 2 below:

$$[Equation\ 2]$$

$$\varepsilon(\%) = \frac{(m_w - m_d)/\rho_w}{(m_w - m_d/\rho_w) + (m_d/\rho_p)} \times 100\%$$

(In the above Equation, $m_w$ is the weight of the wetted hollow fiber membrane bundle with IPA infiltrated into the pores, $m_d$ is the weight of the dried hollow fiber membrane bundle, $\rho_w$ is the density of IPA (0.786 g/cm$^3$), and $\rho_m$ is the density of the double-layer hollow fiber membrane of the present invention (1.37 g/cm$^3$).)

Referring to FIG. 7, the porosity of the double-layer hollow fiber membrane was determined through the following steps: measuring the mass of the dried membrane; immersing the dried membrane in IPA; and measuring the mass of the wetted membrane after immersion.

The porosity of the double-layer hollow fiber membranes prepared in Preparation Examples 4-1 to 4-6, as calculated using Equation 2, is shown in Table 2 below:

[Table 2]

| | First Double-Layer Hollow Fiber Membrane | Second Double-Layer Hollow Fiber Membrane | Third Double-Layer Hollow Fiber Membrane | Fourth Double-Layer Hollow Fiber Membrane | Fifth Double-Layer Hollow Fiber Membrane | Sixth Double-Layer Hollow Fiber Membrane |
|---|---|---|---|---|---|---|
| Porosity (%) | 91.51190945 | 92.01927 | 92.95441 | 92.49298 | 92.24977 | 92.2798 |

**Experimental Example 3: Measurement of Pure water permeability (PWP) of Double-Layer Hollow Fiber Membranes**

The pure water permeability of the first to sixth double-layer hollow fiber membranes prepared in Preparation Examples 4-1 to 4-6 was evaluated using a filtration apparatus.

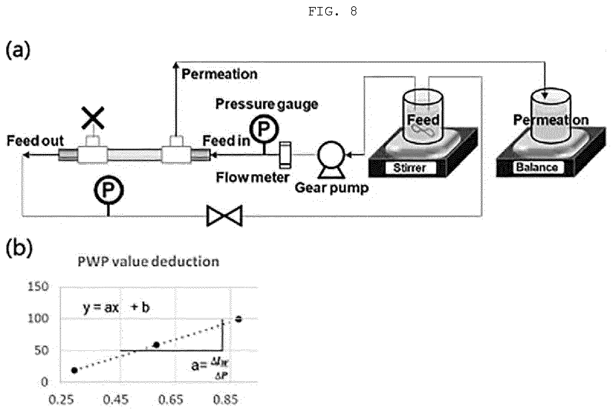

Referring to FIG. 8(a), the filtration apparatus used to measure the pure water permeability of the mini-module fabricated in Preparation Example 5 includes a feed unit, a gear pump, a filtration unit, and a pressure gauge.

4 L of deionized water (DI-water) was prepared in the feed unit, and the feed unit was connected to the mini-modules to allow the water to circulate through the hollow fiber section of the mini-module.

To stabilize the hollow fiber membrane of the mini-module prior to measurement, the transmembrane pressure (TMP), defined as TMP = (feed pressure ($P_{feed-in}$) + concentrate pressure ($P_{feed-out}$))/2, was adjusted to 0.9 bar. The filtration unit was then closed, and deionized water was circulated for 30 minutes to stabilize the hollow fiber membrane.

After stabilization, the filtration unit capable of feeding the second layer of the mini-module was opened in only one direction and connected to a beaker to collect the permeate. Following the connection, the zero point of the pressure gauge was adjusted, and the gear pump was regulated to maintain the flow rate of deionized water between 50 ml/min and 80 ml/min. While maintaining transmembrane pressures of 0.3 bar, 0.6 bar, and 0.9 bar, deionized water was circulated for 40 minutes, and the volume of permeated water was recorded at 1-minute intervals.

Referring to FIG. 8(b), the pure water permeability can be calculated using Equation 3 below. The pure water permeability was calculated based on the measured values averaged over 10 minutes under stabilized conditions.

$$[\text{Equation 3}]$$

$$\text{Pure Water Permeability} = \frac{\triangle J_w}{\triangle P}$$

(In the above Equation, $J_w$ is the flow rate of water per unit area (L·m$^{-2}$·h$^{-1}$), and $\triangle P$ is the transmembrane pressure (TMP, bar).)

The pure water permeability of the double-layer hollow fiber membranes prepared in Preparation Examples 4-1 to 4-6, as calculated using Equation 3, is shown in Table 3 below:

[Table 3]

|  | First Double-Layer Hollow Fiber Membrane | Second Double-Layer Hollow Fiber Membrane | Third Double-Layer Hollow Fiber Membrane | Fourth Double-Layer Hollow Fiber Membrane | Fifth Double-Layer Hollow Fiber Membrane | Sixth Double-Layer Hollow Fiber Membrane |
|---|---|---|---|---|---|---|
| **PWP** | 217.46 | 219.91 | 173.49 | 246.56 | 168.07 | 353.18 |
| $r^2$ | 0.9999 | 0.9461 | 0.9400 | 0.9991 | 0.9204 | 1 |

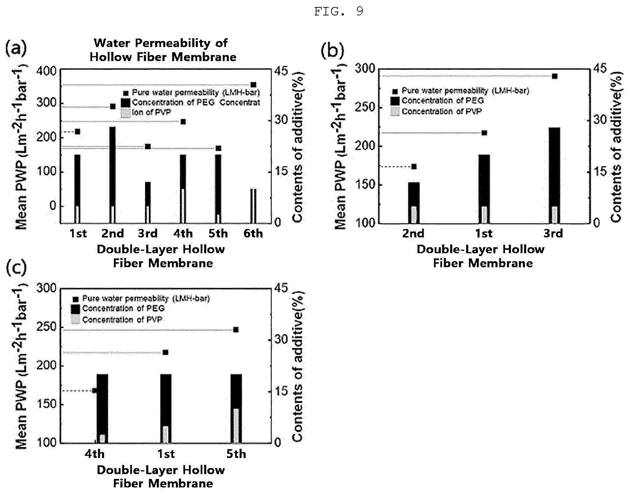

Referring to FIG. 9, it was confirmed that the water permeability of the first to sixth double-layer hollow fiber membranes prepared in Preparation Examples 4-1 to 4-6 increased with the increasing content of pore-forming agents, PVP and PEG, included in the first and second spinning solutions. However, it was observed that the PEG content had a greater effect on the change in water permeability than the PVP content (FIG. 9(b)). This indicates that the water permeability is proportional to the total content of pore-forming agents included in the first and second spinning solutions, although the extent of the increase varies depending on the type of pore-forming agent.

Furthermore, the water permeability of the first to sixth double-layer hollow fiber membranes prepared in Preparation Examples 4-1 to 4-6 was found to fall within the range of 150 LMH ·bar$^{-1}$ to 300 LMH·bar$^{-1}$, which is suitable for use as separation membranes for hemodialysis, hemofiltration, or hemodiafiltration.

**Experimental Example 4: Measurement of Bovine serum albumin (BSA) Leakage of Double-Layer Hollow Fiber Membranes**

The bovine serum albumin (BSA) leakage of the first to sixth double-layer hollow fiber membranes prepared in Preparation Examples 4-1 to 4-6 was evaluated using a filtration apparatus.

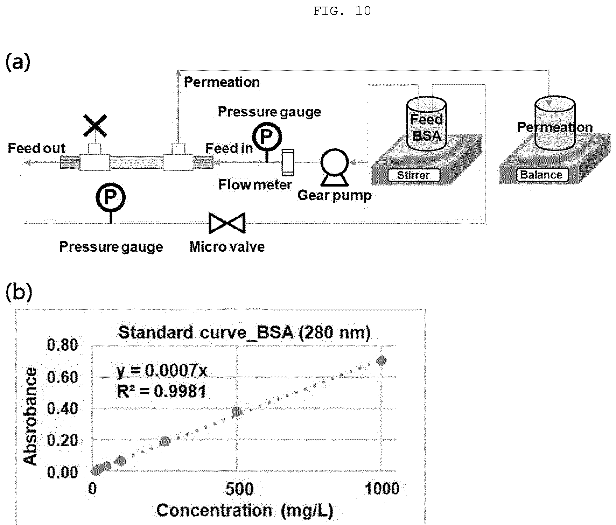

Referring to Fig. 10(a), the BSA leakage was measured using the same filtration apparatus as in Experimental Example 3, except that 500 mL of a 1,000 ppm BSA solution was prepared in the feed unit instead of deionized water.

To stabilize the hollow fiber membrane of the mini-module, the transmembrane pressure (TMP), defined as TMP = (feed pressure ($P_{feed-in}$) + concentrate pressure ($P_{feed-out}$))/2, was adjusted to 0.9 bar. The filtration unit was then closed, and deionized water was circulated for 60 minutes to stabilize the hollow fiber membrane.

After stabilization, the filtration unit capable of feeding the second layer of the mini-module was opened in only one direction and connected to a beaker to collect the permeate. Following the connection, the gear pump was adjusted to maintain a transmembrane pressure of 0.8 bar and to regulate the flow rate of a BSA solution with a concentration of 1,000 ppm between 50 ml/min and 80 ml/min. During circulation of the BSA solution, the permeate was collected at 10-minute intervals over a period of 30 minutes. At each time point, 10 to 15 ml of solution was sampled from both the feed and filtration units.

Referring to FIG. 10(b), the bovine serum albumin (BSA) leakage was calculated using Equation 4 below. The BSA leakage was determined by measuring the UV-vis spectrum and reading the absorbance at a wavelength of 280 nm, from which the concentration was obtained by comparing with a pre-established calibration curve.

[Equation 4]

$$\text{BSA Leakage (\%)} = 100 \times \frac{C_P}{C_F}$$

(In the above Equation, $C_p$ is the concentration of the permeate solution (ppm), and $C_f$ is the concentration of the feed solution (ppm).)

The bovine serum albumin (BSA) leakage of the first to sixth double-layer hollow fiber membranes prepared in Preparation Examples 4-1 to 4-6, as calculated using Equation 4, is shown in Table 4 below:

[Table 4]

|  | First Double-Layer Hollow Fiber Membrane | Second Double-Layer Hollow Fiber Membrane | Third Double-Layer Hollow Fiber Membrane | Fourth Double-Layer Hollow Fiber Membrane | Fifth Double-Layer Hollow Fiber Membrane | Sixth Double-Layer Hollow Fiber Membrane |
|---|---|---|---|---|---|---|
| BSA Leakage | 0.28 % | 1.74 % | 0.03 % | 1.52 % | 1.55 % | 2.78 % |

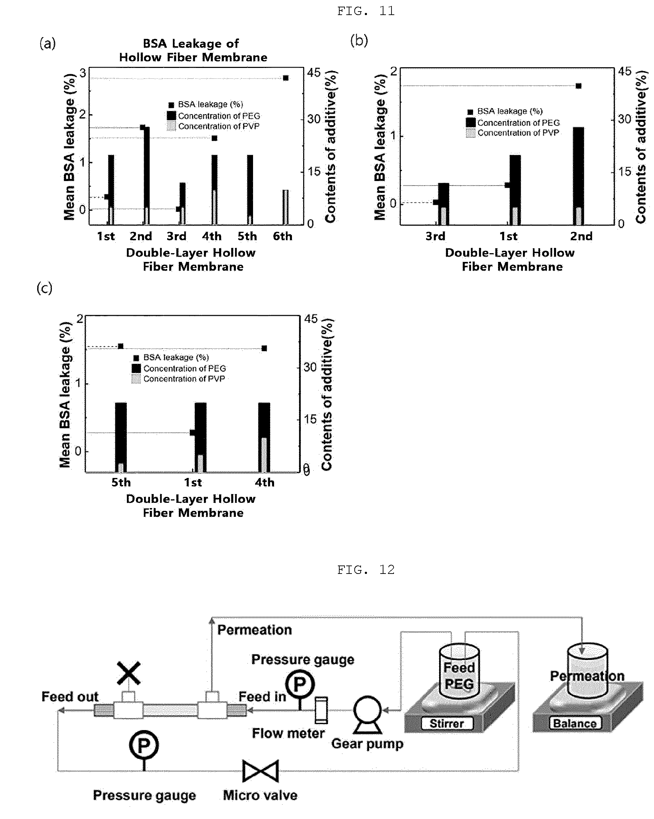

Referring to FIG. 11(a) to (c), it was confirmed that the bovine serum albumin (BSA) leakage of the first to sixth double-layer hollow fiber membranes prepared in Preparation Examples 4-1 to 4-6 was affected by the PVP in the first spinning solution and the PEG in the second spinning solution. In addition, in the case of the fifth double-layer hollow fiber membrane prepared using the first-third spinning solution with a low PVP content, the solvent in the first--third spinning solution dissociates more slowly, resulting in a reduced polymer solidification rate. As a result, the size of the second pores in the second layer of the double-layer hollow fiber membrane increases, potentially leading to an increase in the bovine serum albumin (BSA) leakage.

Moreover, referring to FIG. 11(b), it was confirmed that the bovine serum albumin (BSA) leakage increased with the increasing content of PEG included in the second spinning solution.

Furthermore, as shown in FIG. 11(c), the BSA leakage was found to vary significantly with the PVP content in the first spinning solution.

In addition, it was confirmed that the first and third double-layer hollow fiber membranes prepared in Preparation Examples 4-1 and 4-3 exhibited a BSA leakage of 1% or less. In contrast, the hollow fiber membranes prepared in the other Preparation Examples showed a BSA leakage exceeding 1%, indicating potential limitations for use.

**Experimental Example 5: Measurement of Molecular Weight Cut-Off (MWCO) of Double-Layer Hollow Fiber Membranes**

The molecular weight cut-off (MWCO) of the first and third double-layer hollow fiber membranes, prepared in Preparation Examples 4-1 and 4-3 and exhibiting a BSA leakage of 1% or less, was evaluated using a filtration apparatus.

Referring to FIG. 12, the molecular weight cut-off (MWCO) was determined using the same filtration apparatus as in Experimental Example 3, except that the feed unit was supplied with PEG solutions at a concentration of 200 ppm (MW: 400 Da, 2 kDa, 10 kDa, 44 kDa, 70 kDa, and 100 kDa) instead of deionized water. Since the MWCO was analyzed by total organic carbon (TOC) measurements, TOC calibration solutions with concentrations of 100 ppm, 80 ppm, 40 ppm, 20 ppm, and 10 ppm were prepared.

To stabilize the hollow fiber membrane of the mini-module, the transmembrane pressure (TMP), defined as TMP = (feed pressure ($P_{feed-in}$) + concentrate pressure ($P_{feed-out}$))/2, was adjusted to 0.9 bar. The filtration unit was then closed, and deionized water was circulated for 60 minutes to stabilize the hollow fiber membrane.

After stabilization, the filtration unit capable of feeding the second layer of the mini-module was opened in only one direction and connected to a beaker to collect the permeate. Following the connection, PEG solutions at a concentration of 200 ppm (MW: 400 Da, 2 kDa, 10 kDa, 44 kDa, 70 kDa, and 100 kDa) were prepared in volumes of 500 mL for each molecular weight. The gear pump was then adjusted to maintain a transmembrane pressure (TMP) of 0.8 bar and a flow rate between 50 and 80 mL/min. After circulating each PEG solution for 15 minutes, 10 to 15 mL of both the permeate and feed solutions were collected, and the circulation was terminated.

Before using PEGs with different molecular weights, the filtration unit was closed, and the feed line was reversed. The filtration apparatus was then washed by circulating deionized water at a flow rate of 80 to 160 ml/min under a TMP of 0.8 bar.

The concentrations of the collected solutions were determined using TOC analysis, and the molecular weight-dependent permeability was calculated using Equation 5 below:

[Equation 5]

$$\frac{dR(d_P)}{d(d_P)} = \frac{1}{d_P \ln \sigma_P \sqrt{2\pi}} exp\left\{-\frac{(\ln \sigma_P - \ln \mu_P)^2}{2(\ln \sigma_P)^2}\right\}$$

(In the above Equation, $\mu_p$ is the effective pore size (Rs = 50 %), $\sigma_p$ is the standard deviation of the effective pore size, $r_s$ (nm) is the Stokes radius of PEG, and $d_P$ is defined as $2r_S$ (= $2 \times 16.73 \times 10^{-3} \times M^{0.557}$) .)

The molecular weight cut-offs of the first and third double-layer hollow fiber membranes prepared in Preparation Examples 4-1 and 4-3, as calculated using Equation 5, are shown in Table 5 below. MWRO (molecular weight retention onset), as shown in Table 5, refers to the molecular weight at which the sieving coefficient of uremic toxins starts to decrease below 90%. MWCO stands for molecular weight cut-off.

[Table 5]

|  | Average Pore Size (Standard Deviation) | MWRO | MWCO |
|---|---|---|---|
| First Double-Layer Hollow Fiber Membrane | 9.30 nm(1.492 nm) | 13,660 Da | 59,821 Da |
| Third Double-Layer Hollow Fiber Membrane | 10.14 nm(1.286 nm) | 10,082 Da | 49,888 Da |

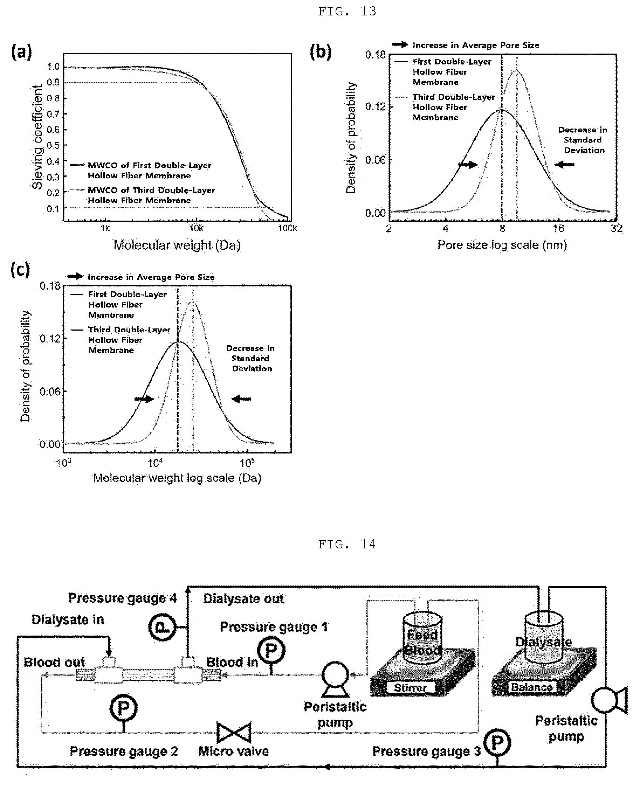

Referring to FIG. 13(a), the MWRO and MWCO values of the first and third double-layer hollow fiber membranes prepared in Preparation Examples 4-1 and 4-3 demonstrate that these double-layer hollow fiber membranes are capable of removing middle-and large-molecular-weight uremic toxins.

As shown in FIG. 13(b), the average pore sizes of the first and third double-layer hollow fiber membranes prepared in Preparation Examples 4-1 and 4-3 fall within the target range of 6 nm to 11 nm, and the standard deviations of the pore sizes are relatively small. Additionally, as shown in FIG. 13(b), although the average pore size of the third double-layer hollow fiber membrane is larger than that of the first double-layer hollow fiber membrane, its more uniform pore distribution contributes to a reduced albumin leakage.

Therefore, it was confirmed that the average pore size, deviation, and pore uniformity of the hollow fiber membrane can be controlled by adjusting the content of PVP included in the first spinning solution and PEG included in the second spinning solution. Moreover it was confirmed that hollow fiber membranes with larger average pore sizes, smaller deviations, and more uniform pores can enhance the ability to remove high-molecular-weight uremic toxins while maintaining low leakage of bovine serum albumin. Through this, it was confirmed that the content of PVP and PEG included in the spinning solution can be adjusted and utilized depending on the intended application of the hollow fiber membrane.

**Experimental Example 5: Measurement of Hemodialysis Performance of Double-layer Hollow Fiber Membrane**

The hemodialysis performance of the first double-layer hollow fiber membrane from Preparation Example 4-1 was confirmed using a filtration apparatus comprising two peristaltic pumps.

Referring to FIG. 14, the hemodialysis performance was evaluated by simultaneously cross-supplying artificial blood and dialysate to the blood section and dialysate section of the mini-module fabricated with the first double-layer hollow fiber membrane using two peristaltic pumps and confirming the clearance of uremic toxins. The artificial blood was prepared by mixing BSA at a concentration of 26 g/L, urea at a concentration of 1 g/L, and creatinine at a concentration of 0.1 g/L into phosphate-buffered saline (PBS).

To stabilize the hollow fiber membrane of the mini-module, the transmembrane pressure (TMP), defined as TMP = (feed pressure ($P_{feed-in}$) + concentrate pressure ($P_{feed-out}$))/2, was adjusted to 0.9 bar. The filtration unit was then closed, and deionized water was circulated for 60 minutes to stabilize the hollow fiber membrane.

After stabilization, artificial blood and dialysate were prepared in separate beakers, and the flow rates of the peristaltic pumps were adjusted to maintain a transmembrane pressure (TMP) of 0 bar (TMP = [($P_1$ + $P_2$)/2 - ($P_3$ + $P_4$)/2] = 0 bar).

Before initiating the flow of artificial blood, 6 ml was collected to measure the concentrations of BSA, urea, and creatinine using UV-vis spectroscopy. Thereafter, 6 ml of solution was collected from both the blood section and the dialysate section at 1-hour intervals over a 4-hour period, and the concentrations were measured. After the 4-hour measurement period, the solutions within the mini-module fabricated using the double-layer hollow fiber membrane, as well as those inside the hollow fiber membrane, were collected from both the blood section and the dialysate section to measure the final volume change.

The permeated amount of uremic toxins was calculated using the measured volume according to Equation 6 below:

[Equation 6]

$$\text{Uremic Toxin Clearance per Unit Area} = \frac{M_U - V_B \times C_{BUt}}{S_M}$$

$$\text{Uremic Toxin Diffusion per Unit Area (from Artificial Blood to Dialysate)} = V_D \times \frac{C_{DUt}}{S_M}$$

$$\text{Uremic Toxin Clearance } C_X (\text{ml} \cdot \text{min}^{-1}) = \frac{A_X}{P_X}$$

Effective Area Ratio between Commercial Membrane Module and Mini-Module: $R_S = \frac{S_{comercial}}{S_{mini}}$

Clearance of Mini-Module Normalized to Surface Area Ratio for Comparison with Clearance of Commercial Membrane Module:

$$C_{mini-X} = C_X \times R_S$$

(In the above Equations:

$M_u$ is the total mass (mg) of a specific uremic toxin dissolved in the artificial blood;

$V_B$ and $V_D$ are the volumes (L) of artificial Blood and Dialysate, respectively;

$C_{BUt}$ and $C_{DUt}$ are the concentrations (ppm) of a specific uremic toxin in the artificial blood and dialysate at a specific time point;

$S_M$ is the effective area (m$^2$) of the membrane in the hollow fiber module;

$C_X$ is the uremic toxin clearance (ml/min);

$A_X$ is the mass (mg/h) of a specific uremic toxin X removed from the artificial blood per hour;

$P_X$ is the average concentration (ppm) of a specific uremic toxin X in the artificial blood over the entire session;

$S_{comercial}$ is the effective area (m$^2$) of the commercial membrane module;

$S_{mini}$ is the effective area (m$^2$) of the mini-module;

$R_S$ is the ratio of the effective area of the commercial membrane module to that of the mini-module; and

$C_{mini-X}$ is the clearance (ml/min) of the mini-module normalized to the surface area ratio for comparison with the clearance of the commercial membrane module.

In the above entire session of $P_X$, one session refers to a 4-hour period during which the hemodialysis filtration test was conducted, with the samples collected at 0, 1, 2, 3, and 4 hours to carry out the test.)

The clearances of uremic toxins and creatinine by the first double-layer hollow fiber membrane prepared in Preparation Example 4-1, as calculated using Equation 6, are shown in Table 6 below:

[Table 6]

|  | BSA Leakage | Urea clearance | Creatinine clearance |
| --- | --- | --- | --- |
| First Double-Layer Hollow Fiber Membrane | 0.14 ± 0.063 % | 257.6 mℓ/min | 574.3 mℓ/min |

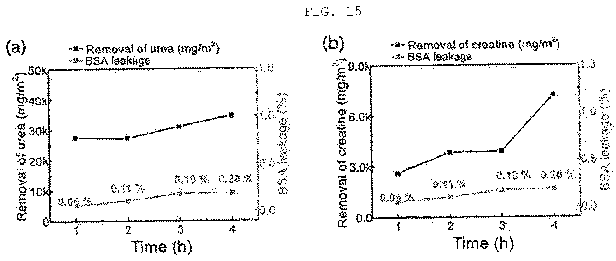

Referring to FIG. 15, it was confirmed that the first double-layer hollow fiber membrane prepared in Preparation Example 4-1 exhibited high clearances of urea and creatinine, while maintaining low leakage of bovine serum albumin.

In conclusion, it was confirmed that the present invention enables the fabrication of a double-layer hollow fiber membrane comprising a hollow core, a first layer having first pores and a second layer having second pores using a first spinning solution containing a polyvinyl-based resin and a polysulfone-based resin and a second spinning solution containing a glycol-based resin and a polysulfone-based resin.

It was confirmed that the first and second pores in the double-layer hollow fiber membrane are interconnected, with the pore size increasing from the first pores toward the second pore. Moreover, SEM analysis confirmed that the second pores are open pores that allow substances to pass through to the outside.

Furthermore, it was confirmed that the present invention enables the fabrication of a double-layer hollow fiber membrane having desired pore size and water permeability by adjusting the content (wt%) of the polyvinyl-based resin included in the first spinning solution and the content of the glycol-based resin included in the second spinning solution.

In addition, it was confirmed that the double-layer hollow fiber membrane manufactured using the first spinning solution and the second spinning solution exhibits low leakage of bovine serum albumin (BSA), while achieving excellent clearances of urea and creatinine, making it suitable for use as a separation membrane for hemodialysis, hemofiltration, or hemodiafiltration.

## Claims

1. A double-layer hollow fiber membrane that surrounds a hollow core through which blood flows and is configured to transfer uremic toxins and waste products in the blood to the outside, the double-layer hollow fiber membrane comprising:

   a first layer having first pores; and
   a second layer connected to the first layer and having second pores that are larger in size than the first pores.

2. The double-layer hollow fiber membrane according to claim 1, wherein the first pores of the first layer are interconnected with and the second pores of the second layer.

3. The double-layer hollow fiber membrane according to claim 1, wherein the second pores of the second layer are in the form of open pores that are connected to the outside.

4. The double-layer hollow fiber membrane according to claim 1, wherein the pore size of the first pores of the first layer ranges from 5 nm to 12 nm, and the pore size of the second pores of the second layer ranges from 0.1 $\mu$m to 1.5 $\mu$.

5. The double-layer hollow fiber membrane according to claim 1, wherein the first pores of the first layer and the second pores of the second layer are formed using different water-soluble pore-forming polymers.

6. The double-layer hollow fiber membrane according to claim 5, wherein the water-soluble pore-forming polymer comprises at least one polyvinyl-based resin selected from the group consisting of polyvinylpyrrolidone (PVP), polyvinylacetate (PVAc), polyvinylalcohol (PVA), and polyethylene-co-vinylacetate (PEVA) or at least one glycol-based resin selected from the group consisting of ethylene glycol (EG), diethylene glycol (DEG), triethylene glycol (TEG), propylene glycol (PG), and polyethylene glycol (PEG).

7. The double-layer hollow fiber membrane according to claim 1, wherein the double-layer hollow fiber membrane has a water permeability of 150 LMH·bar$^{-1}$ to 300 LMH·bar$^{-1}$.

8. The double-layer hollow fiber membrane according to claim 1, wherein the double-layer hollow fiber membrane is used as a separation membrane for hemodialysis, hemofiltration, or hemodiafiltration to filter uremic toxins and waste products from blood.

9. The double-layer hollow fiber membrane according to claim 8, wherein the uremic toxins and waste products in the blood comprise at least one selected from the group consisting of urea, uric acid, beta-2 microglobulin, lambda free light chain, kappa free light chain, creatinine, hippuric acid, indoxyl sulfate, p-cresol, oxalates, guanidines, phenol, and homocysteine.

10. A method for manufacturing a double-layer hollow fiber membrane, the method comprising the steps of:

preparing a first spinning solution by dissolving a first water-soluble pore-forming polymer and a polysulfone-based resin in a solvent;
preparing a second spinning solution by dissolving a second water-soluble pore-forming polymer and a polysulfone-based resin in a solvent;
supplying a bore solution, the first spinning solution, and the second spinning solution to a triple nozzle;
spinning the bore solution, the first spinning solution, and the second spinning solution into a coagulation bath, taking up the spun fiber using a winder to form a hollow fiber membrane;
gelling the resulting hollow fiber membrane in a water bath and washing the resulting hollow fiber membrane in a washing bath containing 80 wt% to 100 wt% of water and 0 wt% to 20 wt% of glycerol; and
drying the resulting hollow fiber membrane in the air at room temperature.

11. The method for manufacturing a double-layer hollow fiber membrane according to claim 10, wherein the bore solution is supplied to a first nozzle, which is at the innermost center of the triple nozzle;

wherein the first spinning solution is supplied to a second nozzle, which is provided on the outer circumference of the first nozzle; and
wherein the second spinning solution is to a third nozzle, which is provided on the outer circumference of the second nozzle.

12. The method for manufacturing a double-layer hollow fiber membrane according to claim 10, wherein the flow rate of the bore solution to the nozzle ranges from 0.3 ml/min to 1.0 ml/min, and the flow rate of the first and second spinning solutions ranges from 0.1 ml/min to 0.5 ml/min.

13. The method for manufacturing a double-layer hollow fiber membrane according to claim 10, wherein the bore solution comprises at least one selected from the group consisting of water, glycerol, N-methyl-2-pyrrolidone, a glycol-based resin, an alcohol-based solvent, and a ketone-based solvent.

14. The method for manufacturing a double-layer hollow fiber membrane according to claim 10, wherein the first the water-soluble pore-forming polymer comprises at least one polyvinyl-based resin selected from the group consisting of polyvinylpyrrolidone (PVP), polyvinylacetate (PVAc), polyvinylalcohol (PVA), and polyethylene-co-vinylacetate (PEVA),

wherein the second water-soluble pore-forming polymer comprises at least one glycol-based resin selected from the group consisting of ethylene glycol (EG), diethylene glycol (DEG), triethylene glycol (TEG), propylene glycol (PG), and polyethylene glycol (PEG), and
wherein the polysulfone-based resin comprises at least one resin selected from the group consisting of polyethersulfone (PES), polysulfone (PSU), and polyphenylenesulfone (PPSU).

15. The method for manufacturing a double-layer hollow fiber membrane according to claim 10, wherein the first spinning solution comprises 1 wt% to 20 wt% of first water-soluble pore-forming polymer, 10 wt% to 20 wt% of polysulfone-based resin, and 65 wt% to 85 wt% of N-methyl-2-pyrrolidone.

16. The method for manufacturing a double-layer hollow fiber membrane according to claim 10, wherein the second spinning solution comprises 5 wt% to 30 wt% of water-soluble pore-forming polymer, 10 wt% to 20 wt% of polysulfone-based resin, and 50 wt% to 80 wt% of N-methyl-2-pyrrolidone.

FIG. 1

Third Nozzle (Second Spinning Solution Section)
Second nozzle (First Spinning Solution Section)
First Nozzle (Bore Solution Section)

FIG. 2

Dual Nozzle

Bore Solution (water/NMP/glycerol=45/45/10)
First Spinning Solution (PES + PVP + NMP)
Second Spinning Solution (PES + PEG +NMP)

Coagulation Bath

Formation of Open Pores

Poly(ethylene glycol) (PEG)

Polyvinylpyrrolidone (PVP)

FIG. 3

FIG. 4

FIG. 5

| Classification | Preparation Example 4-1 | Preparation Example 4-2 | Preparation Example 4-3 | Preparation Example 4-4 | Preparation Example 4-5 | Preparation Example 4-6 |
|---|---|---|---|---|---|---|
| X 200 | | | | | | |
| X 1,500 | | | | | | |

FIG. 6

Cross-Sectional View of Hollow Fiber Membrane

Cross-Section of Outer Membrane Side

Cross-Section of Inner Membrane Side

Membrane Surface of Outer Membrane Side

Membrane Surface of Inner Membrane Side

FIG. 7

Measurement of Mass of Dried Membrane

Immersion in IPA for 24 Hours

Measurement of Mass of Wetted Membrane

FIG. 8

(a)

(b)

PWP value deduction

FIG. 9

(a) Water Permeability of Hollow Fiber Membrane

(b)

(c)

FIG. 10

(a)

(b)

Standard curve_BSA (280 nm)

y = 0.0007x
R² = 0.9981

FIG. 11

(a)

**BSA Leakage of
Hollow Fiber Membrane**

(b)

(c)

FIG. 12

FIG. 13

FIG. 14

FIG. 15

## INTERNATIONAL SEARCH REPORT

| International application No. |
|---|
| **PCT/KR2023/017026** |

| **A.** | **CLASSIFICATION OF SUBJECT MATTER** |
|---|---|

**B01D 69/08**(2006.01)i; **B01D 69/12**(2006.01)i; **B01D 69/10**(2006.01)i; **A61M 1/16**(2006.01)i; **B01D 67/00**(2006.01)i; **B01D 71/68**(2006.01)i

According to International Patent Classification (IPC) or to both national classification and IPC

| **B.** | **FIELDS SEARCHED** |
|---|---|

Minimum documentation searched (classification system followed by classification symbols)

B01D 69/08(2006.01); A61M 1/14(2006.01); A61M 1/18(2006.01); B01D 61/24(2006.01); B01D 63/02(2006.01); B01D 71/68(2006.01); B32B 5/24(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 혈액투석 (hemodialysis), 중공사 (hollow fiber), 멤브레인 (membrane)

| **C.** | **DOCUMENTS CONSIDERED TO BE RELEVANT** |
|---|---|

| Category* | Citation of document, with indication, where appropriate, of the relevant passages | Relevant to claim No. |
|---|---|---|
| X | KR 10-2016-0119190 A (GAMBRO LUNDIA AB) 12 October 2016 (2016-10-12)<br>See paragraphs [0001], [0028] and [0043]; and figures 1 and 8. | 1-4,7-9 |
| A | | 5-6,10-16 |
| X | KR 10-2016-0090535 A (INDUSTRY-ACADEMIC COOPERATION FOUNDATION GYEONGSANG NATIONAL UNIVERSITY) 01 August 2016 (2016-08-01)<br>See claims 7-17; and paragraphs [0041]-[0049]. | 10-13,15-16 |
| A | KR 10-0416135 B1 (KOLON CORPORATION) 07 May 2004 (2004-05-07)<br>See entire document. | 1-16 |
| A | JP 2010-214124 A (MEMBRANA GMBH) 30 September 2010 (2010-09-30)<br>See entire document. | 1-16 |

| ✓ Further documents are listed in the continuation of Box C. | ✓ See patent family annex. |
|---|---|

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **13 February 2024** | **14 February 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

| International application No. |
| --- |
| **PCT/KR2023/017026** |

## C.    DOCUMENTS CONSIDERED TO BE RELEVANT

| Category* | Citation of document, with indication, where appropriate, of the relevant passages | Relevant to claim No. |
| --- | --- | --- |
| A | KR 10-2007-0049196 A (ASAHI KASEI MEDICAL CO., LTD.) 10 May 2007 (2007-05-10)<br>See entire document. | 1-16 |

Form PCT/ISA/210 (second sheet) (July 2022)

## INTERNATIONAL SEARCH REPORT
### Information on patent family members

| International application No. |
| --- |
| **PCT/KR2023/017026** |

| Patent document cited in search report | | | Publication date (day/month/year) | Patent family member(s) | | | Publication date (day/month/year) |
| --- | --- | --- | --- | --- | --- | --- | --- |
| KR | 10-2016-0119190 | A | 12 October 2016 | CN | 105722582 | A | 29 June 2016 |
| | | | | CN | 111545068 | A | 18 August 2020 |
| | | | | CN | 115445438 | A | 09 December 2022 |
| | | | | EP | 3102312 | A1 | 14 December 2016 |
| | | | | EP | 3102312 | B1 | 26 September 2018 |
| | | | | EP | 3427814 | A1 | 16 January 2019 |
| | | | | EP | 3427814 | B1 | 03 August 2022 |
| | | | | JP | 2017-507706 | A | 23 March 2017 |
| | | | | JP | 2020-039952 | A | 19 March 2020 |
| | | | | JP | 6636437 | B2 | 29 January 2020 |
| | | | | JP | 6924253 | B2 | 25 August 2021 |
| | | | | KR | 10-2422691 | B1 | 18 July 2022 |
| | | | | US | 10661230 | B2 | 26 May 2020 |
| | | | | US | 11273417 | B2 | 15 March 2022 |
| | | | | US | 11666869 | B2 | 06 June 2023 |
| | | | | US | 2017-0165615 | A1 | 15 June 2017 |
| | | | | US | 2020-0316533 | A1 | 08 October 2020 |
| | | | | US | 2022-0152563 | A1 | 19 May 2022 |
| | | | | US | 2023-0271146 | A1 | 31 August 2023 |
| | | | | WO | 2015-118046 | A1 | 13 August 2015 |
| KR | 10-2016-0090535 | A | 01 August 2016 | None | | | |
| KR | 10-0416135 | B1 | 07 May 2004 | None | | | |
| JP | 2010-214124 | A | 30 September 2010 | CN | 100448516 | C | 07 January 2009 |
| | | | | CN | 1921929 | A | 28 February 2007 |
| | | | | EP | 1718400 | A1 | 08 November 2006 |
| | | | | EP | 1718400 | B1 | 16 April 2008 |
| | | | | JP | 2007-522851 | A | 16 August 2007 |
| | | | | JP | 4587079 | B2 | 24 November 2010 |
| | | | | US | 2008-0000828 | A1 | 03 January 2008 |
| | | | | US | 8302781 | B2 | 06 November 2012 |
| | | | | WO | 2005-082502 | A1 | 09 September 2005 |
| KR | 10-2007-0049196 | A | 10 May 2007 | CN | 101035576 | A | 12 September 2007 |
| | | | | CN | 101035576 | B | 12 January 2011 |
| | | | | EP | 1790364 | A1 | 30 May 2007 |
| | | | | EP | 1790364 | B1 | 12 September 2012 |
| | | | | JP | 4992106 | B2 | 08 August 2012 |
| | | | | US | 2008-0237127 | A1 | 02 October 2008 |
| | | | | US | 8828225 | B2 | 09 September 2014 |
| | | | | WO | 2006-024902 | A1 | 09 March 2006 |

Form PCT/ISA/210 (patent family annex) (July 2022)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 101810470

- KR 1020050078748

**Non-patent literature cited in the description**

- Current Status of Renal Replacement Therapies in Korea. Korean Society of Nephrology, 2023